# EUROPEAN PATENT SPECIFICATION

(11) **EP 3 720 547 B1**
(45) Date of publication and mention of the grant of the patent: **28.05.2025**
(21) Application number: 18836730.4
(22) Date of filing: 04.12.2018
(51) Int. Cl.: A61N 1/36, A61F 2/82

(54) **PHYSIOLOGICALLY ACTIVE IMPLANTABLE BIOMATERIALS HAVING ENGINEERED FUNCTIONAL SURFACES**
PHYSIOLOGISCH AKTIVE IMPLANTIERBARE BIOMATERIALIEN MIT VERÄNDERTEN FUNKTIONELLEN OBERFLÄCHEN
BIOMATÉRIAUX IMPLANTABLES PHYSIOLOGIQUEMENT ACTIFS COMPORTANT DES SURFACES FONCTIONNELLES MODIFIÉES

(30) Priority: 05.12.2017 US 201762594842 P
(43) Date of publication of application: 14.10.2020
(73) Proprietor: Vactronix Scientific, LLC, Fremont, California 94538 (US)
(72) Inventor: PALMAZ, Julio C., Napa, CA 94558 (US); PALMAZ, Christian G., Fremont, CA 94538 (US); CARPENTER, Scott P., Fremont, CA 94555 (US)
(74) Representative: FRKelly
(86) International application number: PCT/US2018/063838
(87) International publication number: WO 2019/113062

(56) References cited:
- WO-A1-2017/070252
- US-A1- 2010 179 449
- US-A1- 2016 038 087
- US-A1- 2016 066 789
- US-A1- 2017 326 355

## Description

### BACKGROUND OF THE INVENTION

The present invention relates generally to implantable medical devices, in particular stents, and more particularly to implantable wireless medical devices having surface topographies suitable for operably coupling to and functionally interfacing with tissue, such as vascular tissue, adjacent to the implantable medical device. Implantable medical devices according to the present invention will be chronically implantable into a mammalian body and have electronic circuitry that is either embedded in the medical device or is formed as an integral part of the medical device and is configured to sense and/or stimulate tissue, such as nerves, adjacent to or in proximity to the situs of the implantable medical device.

As described by Chow, E., et al, Evaluation of Cardiovascular Stents as Antennas for Implantable Wireless Applications, IEEE Transactions on Microwave Theory and Techniques, 57:10 pp.2523-2532 (Oct. 2009), wireless active stents act both as the package substrate and as the radiating structure. Chow, et al made a non-operational prototype by surface mounting a MEMS capacitive sensor and an application-specific integrated circuit with an onboard transmitter, wireless powering and sensor interface circuits onto a nitinol ZILVER 635 (Cook Medical) self-expanding stent. To test the configuration, an external power source wired to the integrated circuit traces was employed. The test configuration was tested both in free space and *in vivo* by implanting the device in a porcine chest cavity at a depth of 3.5 cm. Using an external horn antenna placed 10 cm distance away from the stent-based transmitter and the power received from the stent-based transmitter were measured, demonstrating the feasibility of using the stent as an active antenna for transmitting signals. Chow, et al. also described in US 9,662,021 wherein a coil stent configured to be disposed in a pulmonary artery is also configured to serve as an antenna for wireless transmission of arterial pressure data. The architecture of a 700 µ x 700 µ Application Specific Integrated Circuit (ASIC) carrying a sensor interface, wireless transmitter, voltage regulator and references, RF powering block and testing pads is also disclosed.

Similarly, Keikhosravy, K. et al. made a stent for monitoring in-stent restenosis in which an integrated circuit is embedded on a platform on the surface of the stent. Keikhosravy, K., et al., On the Use of Smart Stents for Monitoring In-Stent Restenosis, 34th Annual International Conference of the IEEE EMBS, San Diego, CA 28 August - 1 September 2012, pp. 3231-3234. The integrated circuit includes a capacitance to frequency converter (CFC) circuit that consisting of an LC oscillator. The inductor of the CFC is integrated on-chip and the capacitance is the capacitance of a pressure sensor capacitor. The stent acts as an inductor, i.e., it acts like an antenna both to receive power and send data to an external reader. *In vitro* testing and simulations demonstrated that 56 µW at about 0.8 GHz frequency cold be delivered to the stent sufficient to operate the integrated circuit mounted on the stent for active monitoring of in-stent restenosis. Moreover, the simulations demonstrated that the received power was sufficient to transmit information with a power density of about 5pW/cm² at the skin surface, which can be retrieved by an external reader.

Recently, Luo, Y, et al reported development of an RF-powered electrothermally active stent with an integrated temperature limiter for wireless hyperthermia treatment. L., Y, et. al, RF-Powered Stent With Integrated Circuit Breaker for Safeguarded Wireless Hyperthermia Treatment, J. Microelectronic Systems, 24:5, pp. 1293-1302 (Oct. 2015).

Park, J., et al., developed a wireless pressure sensor that was epoxied to a polymer stent. In vitro testing validated a relationship between pressure and resonance frequency from the pressure sensor.

Oxley, et al. in WO 2017/070252 describe a stent with a plurality of wired electrodes coupled to a stent. The electrodes sense and/or stimulate neurons under either wired or wireless control of an external controller unit. The stent described by Oxley, et al. employs an implanted chip that is mounted on the stent and includes circuitry for signal amplification, signal multiplexing and power and data transmission.

Hunter, in US 2016/0038087 describes a stent having a plurality of openings that pass either entirely through or partially into a thickness of the stent. Various sensors, such as blood flow sensor, pressure sensor, position sensor, pH sensor, are positioned within the openings. The sensors are interrogated by an external control unit and are powered by a power scavenger circuit that generates power from mechanical movement.

US 2016/066789 A1 represents further background art.

Each of the foregoing references represent the general state of the art.

For purposes of clarity, but without intent to be limited to a particular structure, the implantable medical device will be referred to as a stent. Stents are well known in the art as radially expandable scaffolds that are typically percutaneously delivered to a situs within a body lumen. Stents may comprise a plurality of structural support members forming a tubular structure having a luminal surface facing the body lumen and an abluminal surface facing the body tissue. The term "physiologically active medical device" is intended to encompass a type of implantable medical devices (or "physiologically active stent") that have embedded electronic circuitry capable of receiving externally applied signals, processing the externally applied signals and responding to the externally applied signals in a predetermined manner. The response by the active stent may be to emit an electrical signal to local tissue adjacent the active stent, transmit sensor data, activate sensor functions, or any such other function to activate active stent functionality. External signals may be applied by an external device that wirelessly communicates with the electronic circuitry in the active stent.

The medical devices or stents of the present invention are particularly well-suited for neurovascular delivery to a situs within the brain where the stent may be in electrical communication with portions of the brain that communicate signals to different sensory or motor portions of the body. For example, the Broca's area of the brain is implicated language production, and in planning and imitating movement and understanding another's movement. It has recently been found that Broca's area appears to act as an intermediary between the temporal cortex, which organizes incoming sensory information, and the motor cortex, which carries out the movements of the mouth. It is known that the M1 horizontal segment of the middle cerebral artery supplies the Broca's area and that decreased perfusion of this vessel or proximal to it causes Broca's aphasia. Placement of the inventive active device in the M1 segment of the middle cerebral artery will allow for both signal sensing and stimulation of the Broca region. Alternatively, the primary motor cortex, which lies posterior and adjacent Broca's region, controls both motor imagery, i.e., motor cognition and motor function, is supplied by both the middle and anterior cerebral arteries, with the middle cerebral artery supplying the lateral primary motor cortex and the anterior cerebral artery supplying the medial primary motor cortex. The homunculus describes the organization of the precentral gyrus of the primary motor cortex, with the lower parts of the body, e.g., legs and feet, receiving motor movement commands from the superior part of the precentral gyrus. The upper parts of the body, e.g., face and arms are innervated by the inferior part of the precentral gyrus. Placement of the inventive active device in the anterior cerebral artery will allow for both signal sensing and stimulation of the corresponding portions of the primary motor cortex.

Thus, the inventive active device has particular use in detecting neurological conditions in neuronal signaling, vascular flow or pressure or the like. The inventive active device also has particular use in neurostimulation as it is capable of delivering electrical signals through onboard electrodes to different regions of the brain accessible through the neurovasculature.

Those skilled in the art will appreciate that the inventive active device also has use in the coronary arteries and may be used for cardio-pacing, for monitoring coronary artery flow and pressure, or for detecting coronary or cardiac conditions and stimulating cardiac tissue. It will further be appreciated that the inventive active device may be employed in other anatomic lumens or organ systems both for detection of conditions and for nerve stimulation.

The implantable medical device is configured to have a surface topography configured to increase surface area contact with adjacent tissue such that electrical contact is made between the implantable medical device and the adjacent tissue. The surface topography may consist of raised projections that are in contact with or penetrate into the adjacent tissue. Preferably, the raised projections are formed integrally with and of the material of the implantable medical device.

The inventive implantable medical devices may be fabricated of polymers, pre-existing conventional wrought metallic materials, such as stainless steel or nitinol hypotubes, or may be fabricated by vacuum deposition of metallic or pseudometallic materials. In accordance with the present invention, it is preferable to fabricate the inventive implantable materials and resulting devices by vacuum deposition of either or both of the base implant material and the chemically and/or physiochemically active features. Vacuum deposition permits a high level of control over many material characteristics and properties of the resulting deposited material and formed device. Vacuum deposition allows for control over the material properties of the deposited material, including, for example, grain size, grain phase, grain material composition, bulk material composition and mechanical properties, such as transition temperatures in the case of a shape memory alloy. Vacuum deposition techniques also allow for precise sub-micron feature formation during fabrication of the resulting device. Features may be formed during deposition both within the thickness of the deposited material and/or on surfaces of the deposited material. For example, multi-layer structures, complex geometrical configurations, feature and material tolerances, such as thickness or geometry, are all advantages of vacuum deposition processing.

In vacuum deposition technologies, materials are formed directly in the desired geometry, *e.g.,* planar, tubular, layered, etc. As is well known in the microelectronics fabrication field, complex layered electronic circuitry, including transistors, diodes, capacitors, amplifier circuits, oscillators, timers, counters, memory, etc. may all be fabricated by microelectronics fabrication techniques that are well known.

The application of microelectronic fabrication techniques to medical device or stent fabrication is still in a nascent stage. Fabrication of medical devices, such as stents, by vacuum deposition techniques has been accomplished, as represented by US 6,379,383 and US 6,357,310, which are hereby incorporated by reference.

In addition to materials and devices that are made of a single metal or metal alloy layer, the inventive devices may be comprised of a layer of biocompatible material or of a plurality of layers of biocompatible materials formed upon one another into a self-supporting multilayer structure because multilayer structures are generally known to increase the mechanical strength of sheet materials, or to provide special qualities by including layers that have special properties such as superelasticity, shape memory, radio-opacity, corrosion resistance etc. A special advantage of vacuum deposition technologies is that it is possible to deposit layered materials and thus films possessing exceptional qualities may be produced (cf., H. Holleck, V. Schier: Multilayer PVD coatings for wear protection, Surface and Coatings Technology, Vol. 76-77 (1995) pp. 328-336). Layered materials, such as superstructures or multilayers, are commonly deposited to take advantage of some chemical, electronic, or optical property of the material as a coating; a common example is an antireflective coating on an optical lens. Multilayers are also used in the field of thin film fabrication to increase the mechanical properties of the thin film, specifically hardness and toughness. Vacuum deposition fabrication of such layered biomaterials is disclosed in US 9,566, 148.

Finally, surface topography features, such as raised features or recessed features, may be made by photolithography as taught by US 9,050,394, or by additive or subtractive vacuum deposition techniques as taught by US 7,736,687.

While there is a growing body of work directed toward creating physiologically active wireless medical devices, the goal of having stents with fully integrally fabricated on-board electronic circuitry, has so-far proven elusive. Nano- and microelectronic technology has been successful in creating extremely small-scale electronic components suitable for use with the present invention. The current state of the art in smart or active medical devices has relied upon coupling, such as by welding or epoxy adhesive, the electronic circuitry to a bedding surface on a stent. Medical device fabrication technology has, heretofore, not deployed manufacturing technologies well-suited to making implantable physiologically active medical devices with integrally fabricated on-board electronics.

### SUMMARY OF THE INVENTION

The present invention is defined by the appended claims and provides a wireless physiologically active implantable stent according to claim 1 and a method of making a wireless physiologically active implantable stent according to claim 10. Embodiments of the invention are defined by the dependent claims.

It is an objective of the present invention to provide an implantable physiologically active medical device that, once implanted to a situs within a mammalian body, is configured to wirelessly communicate with an external telemetry device.

It is another objective of the present invention to provide an implantable physiologically active stent having integrally formed on-board electronic circuitry configured to communicate with tissue adjacent the physiologically active stent to sense or detect a condition or conditions within the body and/or to issue a stimulating signal to nerves or other tissue proximate to the situs of the implanted physiologically active stent.

It is still another objective of the present invention that the on-board integrated electronic circuity be fabricated as an integral part of the stent structure during fabrication of the stent structure and not formed as a separate component added to a pre-formed stent-structure.

It is yet another objective of the present invention that the on-board integrated electronics include an LC circuit configured to wirelessly communicate with an external control device outside the body. The external control device will periodically energize the capacitive element in the LC circuit in the implanted physiologically active device.

It is a further objective of the present invention that the on-board integrated electronics in the physiologically active stent include an LC circuit, a transmitter, a power supply, and an analog-digital converter.

It is yet another objective of the present invention to provide a method for making an implantable medical device with on-board integrated electronics configured to wireless communicate with an external control unit to detect a condition in the body and/or stimulate tissue in the region of the implanted medical device.

The methods, systems, and apparatuses are set forth in part in the description which follows, and in part will be obvious from the description, or can be learned by practice of the methods, apparatuses, and systems. The advantages of the methods, apparatuses, and systems will be realized and attained by means of the elements and combinations particularly pointed out in the appended claims. It is to be understood that both the foregoing general description and the following detailed description are exemplary and explanatory only and are not restrictive of the methods, apparatuses, and systems, as claimed.

### BRIEF DESCRIPTION OF THE FIGURES

In the accompanying figures, like elements are identified by like reference numerals among the several preferred embodiments of the present invention.
FIG. 1 is partial cross-sectional diagrammatic view of a stent or medical device illustrating integrally formed electronic circuitry in accordance with the present invention.
FIG. 2 is a process flow chart outlining the method of making the inventive physiologically active stent or medical device.

### DETAILED DESCRIPTION OF THE PREFERRED EMBODIMENTS

The foregoing and other features and advantages of the invention are apparent from the following detailed description of exemplary embodiments, read in conjunction with the accompanying drawings. The detailed description and drawings are merely illustrative of the invention rather than limiting, the scope of the invention being defined by the appended claims and equivalents thereof.

The wireless physiologically active implantable medical device 10 of the present invention integrally and substantially monolithically combines a stent 12 with microelectronic components 14 and electrodes 26. The stent 12 is preferably formed by vacuum depositing a stent forming material onto a substrate. The stent-forming material is preferably a metal suitable for use as an antenna capable of transmitting and receiving radiofrequency or other electromagnetic signals across body tissue. Of course, because it is implantable, stent 12 must also be biocompatible. A shape memory metal, such as Nitinol, is well suited both as a stent material and as an antenna. Binary, ternary, quaternary or other metal alloys may be employed as the stent-forming (or device-forming) material. Non-limiting examples include NiTi, NiTiCo, NiTiPt, NiTiPd, NiTiHf, NiTiZr, NiTiAu, NiTiCr, NiTiW, NiTiCoZr, or NiTiCuPd.

Stent 12 is formed having a plurality of structural members and preferably formed with at least one recess in at least one structural member of the plurality structural members. The recess may be formed during the vacuum deposition of the stent material or formed by post-processing techniques, such as photolithography. The microelectronic components 14 and electrodes 26 are then formed *in situ* within the recesses by known microelectronic fabrication techniques used to manufacture such devices, including, for example, physical vapor deposition of the individual sub-layers and sub-components of the microelectronic components and patterning by photolithography and etching the sub-layers and sub-components of the microelectronic components 14 or electrodes 26.

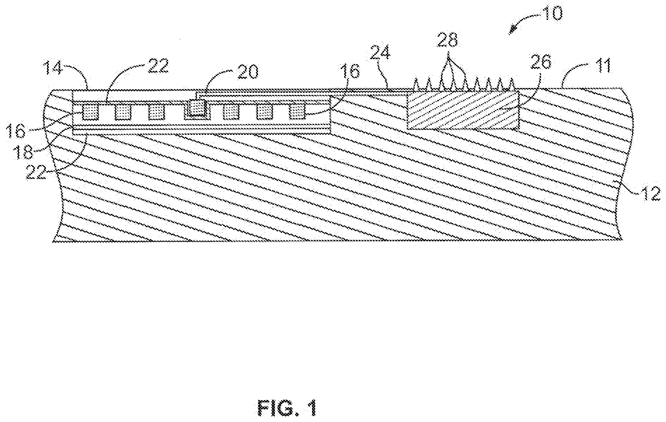

For exemplary purposes only, Fig. 1 depicts microelectronic component 14 as an LC circuit comprising a plurality of inductors 16, capacitor layers 18, 20 and dielectric layers 22 that isolate the LC circuit.

Microelectronic component 14 may be configured as an LC circuit, an amplifier, a transmitter, filter, tuner, power supply, an analog-digital converter, memory, computer, sensor or any such other microelectronic component 14 as is capable of being formed integrally and substantially monolithically within the plurality of recesses in stent 12. Those skilled in the art of microelectronic fabrication will understand that the plurality of recesses function as packaging for the microelectronic component 14.

Similarly, electrode 26 is preferably formed by vacuum depositing metal capable of acting as an electrode into electrode recesses in the stent, and depositing onto stent 12 an electrically conductive layer 24 electrically coupling microelectronic component 14 to electrode 26. In this arrangement, electrical energy transmitted by microelectronic component 14 will be discharged by electrode 26 to tissue adjacent the stent 12.

To further facilitate electrically coupling of the stent 12, namely electrodes 26, to the tissue adjacent the stent 12, raised surface topographical features 28 may be provided on the surface of the electrodes 26 which will act as micro-needles and engage the tissue allowing for better electrical contact between the electrodes 26 and the tissue. In some embodiments, each micro-needle has a height of less than or equal to about 10.0 microns.

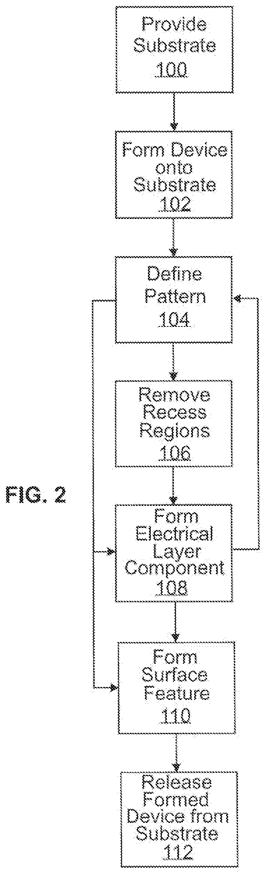

Fig. 2 is a process flow diagram depicting a method for fabricating the inventive physiologically active medical device 12 in accordance with the present invention.

A substrate is provided 100 and a device forming material, preferably metal, is vacuum deposited onto the substrate 102. Once the device forming material is deposited, it is patterned 104 with a pattern corresponding to the recesses to be formed in a surface of the device forming material. As is well known in photolithography, a photoresist is applied to the surface of the device forming material, and a positive or negative mask is applied to the photoresist and the photoresist is exposed to light through the mask. Alternatively, a positive or negative photoresist may be employed. The exposed photoresist is then developed to remove the exposed photoresist thereby uncovering patterned regions of the device forming material to be removed. Subsequent removal 106 of portions of the patterned regions of the device forming material defines the plurality of recesses in the surface of the device forming material. Removal may be by wet or dry etching, ablation, or laser cutting, or other equivalent processes that yield high resolution recess features in the surface of the device forming material.

With the plurality of recesses formed in the surface of the device, conventional microelectronic fabrication techniques may be followed to deposit and form electrical component layers 108 or traces within the recess along with forming the electrodes in the electrode recesses. The electrical component layers 108, traces and/or the electrodes may be also be formed by 3D printing into the plurality of recesses. Once the microelectronic circuitry and electrodes are formed, desired surface features on the electrodes, such as the tissue contact projections, may be formed 110 by deposition, photolithography and/or 3D printing or other processes adapted to form high resolution features on the surface of the device forming material.

Once the device is fully formed, it is released 112 from the substrate and any post-processing steps, such as validation, quality control, quality assurance, or the like may be undertaken on the device.

Finally, it will be understood that the wireless physiologically active medical device of the present invention may be formed as tubular cylinder, as with conventional radially expandable stents, or may be formed as a planar material that is capable of being coiled or folded for delivery and implantation into the body in its final device shape. The final device shape may cover all or a portion of the circumference of the body lumen at the situs of implantation.

Vacuum deposition onto both cylindrical and planar substrates is known in the art, as exemplified by US 6,379,383 and US 6,357,310. Similarly, 3D printing onto cylindrical surfaces is also known in the art, as exemplified by WO 2011/011818. 3D printing onto planar substrates is well known.

While the invention has been described in connection with various embodiments, it will be understood that the invention is capable of further modifications. This application is intended to cover any variations, uses or adaptations of the invention following, in general, the principles of the invention, and including such departures from the present disclosure as, within the known and customary practice within the art to which the invention pertains. The foregoing notwithstanding, the invention is solely defined by the appended claims.

## Claims

1. A wireless physiologically active implantable stent (12),
comprising a scaffold configured to be delivered to and implanted at a situs in a mammalian body, the scaffold comprising a plurality of structural supports and at least one electronic circuit (14) configured to electrically couple to tissue proximate the situs in a mammalian body,
wherein the at least one electronic circuit (14) is integrally formed as part of the at least one structural support of the plurality of structural supports, and the at least one structural support of the plurality of structural supports further comprises at least one integrally formed region electrically coupling the at least one electronic circuit (14) to tissue proximate the situs in a mammalian body, wherein the scaffold is the stent (12) and the plurality of structural supports form a tubular structure of the stent (12).

2. The wireless physiologically active implantable stent (12) of Claim 1, wherein at least some structural supports of the plurality of structural supports further include at least one recess in an abluminal surface of the at least some structural supports of the plurality of structural supports.

3. The wireless physiologically active implantable stent (12) of Claim 2, wherein the at least one electronic circuit (14) is integrally formed within the at least one recess.

4. The wireless physiologically active implantable stent (12) of Claim 2, wherein the at least one electronic circuit (14) further comprises an integrated circuit.

5. The wireless physiologically active implantable stent (12) of Claim 3, wherein the at least one integrally formed region configured to electrically couple the at least one electronic circuit (14) to tissue at the situs further comprises a raised topography comprising a plurality of micro-needles (28);
wherein, optionally, the plurality of micro-needles (28) is configured to receive electrical signals from the electronic circuit (14) and communicate the electrical signals to tissue in the mammalian body; or
wherein, optionally, each micro-needle of the plurality of micro-needles (28) has a height of less than or equal to about 10.0 microns.

6. The wireless physiologically active implantable stent (12) of Claim 3, wherein the at least one electronic circuit (14) is configured as at least one of an antenna, a transmitter, a power source and/or an electrode (26); wherein, optionally, the stent (12) is an antenna operably coupled to the at least one electronic circuit (14).

7. The wireless physiologically active implantable stent (12) of Claim 4, wherein the integrated circuit further comprises an LC circuit; or
further comprising at least one electrode in electrical communication with the integrated circuit.

8. The wireless physiologically active implantable stent (12) of Claim 3, further comprising at least one electrode in electrical communication with the at least one electronic circuit (14); or wherein the at least one electronic circuit (14) is positioned within a width and depth of the at least one structural support of the plurality of structural supports.

9. The wireless physiologically active implantable stent (12) of Claim 1, wherein each
structural support of the plurality of structural supports has a thickness less than or equal to 75 microns;
wherein, optionally, each structural support of the plurality of structural supports has a width less than or equal to 75 microns.

10. A method of making a wireless physiologically active implantable stent (12) according to any of claims 1-9, comprising the steps of:
a. Providing a substrate 100 for forming the wireless physiologically active implantable stent (12);
b. Vacuum depositing a device forming material onto the substrate (102);
c. Masking portions of the deposited device forming material to define recesses regions to be formed on the device forming material (104);
d. Forming recesses in a surface of the device forming material (106);
e. Depositing at least one electrical component layer of a plurality of electrical component layers into the recesses (108); and
f. Forming surface features on a surface of at least some of the electrical component layers of the plurality of electrical component layers (110).

11. The method of Claim 10, wherein step b further comprises the step of depositing a shape memory metal.

12. The method of Claim 11, further comprising the step of depositing an electrically conductive layer coupling at least one electrical component layer of the plurality of electrical component layers to the device forming material.

13. The method of Claim 12, further comprising the step of depositing an electrically conductive layer coupling at least one electrical component layer of the plurality of electrical component layers to another electrical component layer.

14. The method of Claim 10, wherein step e further comprises 3D printing at least one electrical component layer of a plurality of electrical component layers into the recesses.

## Patentansprüche

1. Drahtloser physiologisch aktiver implantierbarer Stent (12), umfassend ein Gerüst, das dazu konfiguriert ist, an eine Stelle in einem Säugetierkörper abgegeben und an dieser implantiert zu werden, wobei das Gerüst eine Vielzahl von Strukturträgern und mindestens eine elektronische Schaltung (14) umfasst, die dazu konfiguriert ist, sich mit Gewebe in der Nähe der Stelle in einem Säugetierkörper elektrisch zu koppeln, wobei die mindestens eine elektronische Schaltung (14) einstückig als Teil des mindestens einen Strukturträgers der Vielzahl von Strukturträgern gebildet ist und der mindestens eine Strukturträger der Vielzahl von Strukturträgern ferner mindestens eine integral gebildete Region umfasst, welche die mindestens eine elektronische Schaltung (14) elektrisch an Gewebe in der Nähe der Stelle in einem Säugetierkörper koppelt, wobei das Gerüst der Stent (12) ist und die Vielzahl von Strukturträgern eine röhrenförmige Struktur des Stents (12) bilden.

2. Drahtloser physiologisch aktiver implantierbarer Stent (12) nach Anspruch 1, wobei mindestens einige Strukturträger der Vielzahl von Strukturträgern ferner mindestens eine Aussparung in einer Abluminalfläche der mindestens einigen Strukturträger der Vielzahl von Strukturträgern beinhalten.

3. Drahtloser physiologisch aktiver implantierbarer Stent (12) nach Anspruch 2, wobei die mindestens eine elektronische Schaltung (14) einstückig innerhalb der mindestens einen Aussparung gebildet ist.

4. Drahtloser physiologisch aktiver implantierbarer Stent (12) nach Anspruch 2, wobei die mindestens eine elektronische Schaltung (14) ferner eine integrierte Schaltung umfasst.

5. Drahtloser physiologisch aktiver implantierbarer Stent (12) nach Anspruch 3, wobei die mindestens eine einstückig gebildete Region, die dazu konfiguriert ist, die mindestens eine elektronische Schaltung (14) an Gewebe an der Stelle elektrisch zu koppeln, ferner eine erhöhte Topographie umfasst, die eine Vielzahl von Mikronadeln (28) umfasst;
wobei optional die Vielzahl von Mikronadeln (28) dazu konfiguriert ist, elektrische Signale von der elektronischen Schaltung (14) zu empfangen und die elektrischen Signale an Gewebe in dem Säugetierkörper zu übermitteln; oder
wobei optional jede Mikronadel der Vielzahl von Mikronadeln (28) eine Höhe kleiner als oder gleich etwa 10,0 Mikrometer aufweist.

6. Drahtloser physiologisch aktiver implantierbarer Stent (12) nach Anspruch 3, wobei die mindestens eine elektronische Schaltung (14) als mindestens eines von einer Antenne, einem Sender, einer Stromquelle und/oder einer Elektrode (26) konfiguriert ist;
wobei der Stent (12) optional eine Antenne ist, die an die mindestens eine elektronische Schaltung (14) wirkgekoppelt ist.

7. Drahtloser physiologisch aktiver implantierbarer Stent (12) nach Anspruch 4, wobei die integrierte Schaltung ferner eine LC-Schaltung umfasst; oder
ferner umfassend mindestens eine Elektrode in elektrischer Verbindung mit der integrierten Schaltung.

8. Drahtloser physiologisch aktiver implantierbarer Stent (12) nach Anspruch 3, ferner umfassend mindestens eine Elektrode in elektrischer Verbindung mit der mindestens einen elektronischen Schaltung (14); oder
wobei die mindestens eine elektronische Schaltung (14) innerhalb einer Breite und Tiefe des mindestens einen Strukturträgers der Vielzahl von Strukturträgern positioniert ist.

9. Drahtloser physiologisch aktiver implantierbarer Stent (12) nach Anspruch 1, wobei jeder Strukturträger der Vielzahl von Strukturträgern eine Dicke kleiner als oder gleich 75 Mikrometer aufweist;
wobei optional jeder Strukturträger der Vielzahl von Strukturträgern eine Breite kleiner als oder gleich 75 Mikrometer aufweist.

10. Verfahren zum Herstellen eines drahtlosen physiologisch aktiven implantierbaren Stents (12) nach einem der Ansprüche 1-9, umfassend die folgenden Schritte:
a. Bereitstellen eines Substrats 100 zum Bilden des drahtlosen physiologisch aktiven implantierbaren Stents (12);
b. Abscheiden eines vorrichtungsbildenden Materials auf dem Substrat (102) unter Vakuum;
c. Maskieren von Abschnitten des abgeschiedenen vorrichtungsbildenden Materials, um Aussparungsregionen zu definieren, die an dem vorrichtungsbildenden Material (104) zu bilden sind;
d. Bilden von Aussparungen in einer Oberfläche des vorrichtungsbildenden Materials (106);
e. Abscheiden mindestens einer Schicht für elektrische Komponenten aus einer Vielzahl von Schichten für elektrische Komponenten in die Aussparungen (108) durch physikalische Gasphasenabscheidung; und
f. Bilden von Oberflächenmerkmalen auf einer Oberfläche mindestens einiger der Schichten für elektrische Komponenten der Vielzahl von Schichten (110) für elektrische Komponenten.

11. Verfahren nach Anspruch 10, wobei der Schritt b ferner den Schritt des Abscheidens eines Formgedächtnismetalls umfasst.

12. Verfahren nach Anspruch 11, ferner umfassend den Schritt des Abscheidens einer elektrisch leitfähigen Schicht, die mindestens eine Schicht für elektrische Komponenten aus der Vielzahl von Schichten für elektrische Komponenten an das vorrichtungsbildende Material koppelt.

13. Verfahren nach Anspruch 12, ferner umfassend den Schritt des Abscheidens einer elektrisch leitfähigen Schicht, die mindestens eine Schicht für elektrische Komponenten aus der Vielzahl von Schichten für elektrische Komponenten an eine andere Schicht für elektrische Komponenten koppelt.

14. Verfahren nach Anspruch 10, wobei Schritt e ferner 3D-Drucken mindestens einer Schicht für elektrische Komponenten einer Vielzahl von Schichten für elektrische Komponenten in die Aussparungen umfasst.

## Revendications

1. Stent implantable physiologiquement actif sans fil (12), comprenant un échafaudage configuré pour être délivré et implanté à un endroit dans un corps de mammifère, l'échafaudage comprenant une pluralité de supports structurels et au moins un circuit électronique (14) configuré pour se coupler électriquement à un tissu à proximité de l'endroit dans un corps de mammifère, dans lequel l'au moins un circuit électronique (14) est formé d'un seul tenant en tant que partie de l'au moins un support structurel de la pluralité de supports structurels, et l'au moins un support structurel de la pluralité de supports structurels comprennent également au moins une région formée d'un seul tenant couplant électriquement l'au moins un circuit électronique (14) à un tissu à proximité de l'endroit dans un corps de mammifère, dans lequel l'échafaudage est le stent (12) et la pluralité de supports structurels forment une structure tubulaire du stent (12).

2. Stent implantable physiologiquement actif sans fil (12) selon la revendication 1, dans lequel au moins certains supports structurels de la pluralité de supports structurels comportent également au moins un évidement dans une surface abluminale des au moins certains supports structurels de la pluralité de supports structurels.

3. Stent implantable physiologiquement actif sans fil (12) selon la revendication 2, dans lequel l'au moins un circuit électronique (14) est formé d'un seul tenant dans l'au moins un évidement.

4. Stent implantable physiologiquement actif sans fil (12) selon la revendication 3, dans lequel l'au moins un circuit électronique (14) comprend également un circuit intégré.

5. Stent implantable physiologiquement actif sans fil (12) selon la revendication 4, dans lequel l'au moins une région formée d'un seul tenant est configurée pour coupler électriquement l'au moins un circuit électronique (14) au tissu au niveau de l'endroit comprend également une topographie surélevée comprenant une pluralité de micro-aiguilles (28) ;
dans lequel, éventuellement, la pluralité de micro-aiguilles (28) est configurée pour recevoir des signaux électriques du circuit électronique (14) et communiquer les signaux électriques au tissu du corps de mammifère ; ou
dans lequel, éventuellement, chaque micro-aiguille de la pluralité de micro-aiguilles (28) a une hauteur inférieure ou égale à environ 10,0 microns.

6. Stent implantable physiologiquement actif sans fil (12) selon la revendication 3, dans lequel l'au moins un circuit électronique (14) est configuré comme au moins un élément parmi une antenne, un émetteur, une source d'alimentation et/ou une électrode (26) ;
dans lequel, éventuellement, le stent (12) est une antenne couplée de manière opérationnelle à l'au moins un circuit électronique (14).

7. Stent implantable physiologiquement actif sans fil (12) selon la revendication 4, dans lequel le circuit intégré comprend également un circuit LC ; ou
comprenant également au moins une électrode en communication électrique avec le circuit intégré.

8. Stent implantable physiologiquement actif sans fil (12) selon la revendication 3, comprenant également au moins une électrode en communication électrique avec l'au moins un circuit électronique (14) ; ou
dans lequel l'au moins un circuit électronique (14) est positionné dans une largeur et une profondeur de l'au moins un support structurel de la pluralité de supports structurels.

9. Stent implantable physiologiquement actif sans fil (12) selon la revendication 1, dans lequel chaque support structurel de la pluralité de supports structurels a une épaisseur inférieure ou égale à 75 microns ;
dans lequel, éventuellement, chaque support structurel de la pluralité de supports structurels a une largeur inférieure ou égale à 75 microns.

10. Procédé de fabrication d'un stent implantable physiologiquement actif sans fil (12) selon l'une quelconque des revendications 1 à 9, comprenant les étapes :
a. de fourniture d'un substrat 100 pour former le stent implantable physiologiquement actif sans fil (12) ;
b. de dépôt sous vide d'un matériau formant un dispositif sur le substrat (102) ;
c. de masquage de parties du matériau de formation de dispositif déposé pour définir des régions d'évidements à former sur le matériau de formation de dispositif (104) ;
d. de formation d'évidements dans une surface du matériau de formation de dispositif (106) ;
e. de dépôt d'au moins une couche de composant électrique parmi une pluralité de couches de composants électriques dans les évidements (108) par dépôt physique en phase vapeur ; et
f. de formation de caractéristiques de surface sur une surface d'au moins certaines des couches de composants électriques de la pluralité de couches de composants électriques (110).

11. Procédé selon la revendication 10, dans lequel l'étape b comprend également l'étape de dépôt d'un métal à mémoire de forme.

12. Procédé selon la revendication 11, comprenant également l'étape de dépôt d'une couche électriquement conductrice couplant au moins une couche de composant électrique de la pluralité de couches de composant électrique au matériau de formation de dispositif.

13. Procédé selon la revendication 12, comprenant également l'étape de dépôt d'une couche électriquement conductrice couplant au moins une couche de composant électrique de la pluralité de couches de composant électrique à une autre couche de composant électrique.

14. Procédé selon la revendication 10, dans lequel l'étape e comprend également l'impression 3D d'au moins une couche de composant électrique d'une pluralité de couches de composants électriques dans les évidements.
